(19) **Europäisches Patentamt / European Patent Office / Office européen des brevets**

(11) **EP 3 797 752 B1**

(12) **EUROPEAN PATENT SPECIFICATION**

(45) Date of publication and mention of the grant of the patent:
**17.07.2024 Bulletin 2024/29**

(21) Application number: **19806791.0**

(22) Date of filing: **21.05.2019**

(51) International Patent Classification (IPC):
*A61J 1/05* (2006.01)    *A61K 9/19* (2006.01)
*A61K 39/395* (2006.01)    *A61K 47/10* (2017.01)
*A61K 47/18* (2017.01)    *A61K 47/26* (2006.01)
*A61P 43/00* (2006.01)    *A61J 1/06* (2006.01)

(52) Cooperative Patent Classification (CPC):
**A61P 43/00; A61K 9/19; A61K 39/395;**
**A61K 47/10; A61K 47/26;** A61J 1/065; A61J 1/1468

(86) International application number:
**PCT/JP2019/020014**

(87) International publication number:
**WO 2019/225568 (28.11.2019 Gazette 2019/48)**

(54) **LYOPHILIZED FORMULATION SEALED IN GLASS CONTAINER**

IN EINEM GLASBEHÄLTNIS VERSCHLOSSENE LYOPHILISIERTE FORMULIERUNG

FORMULATION LYOPHILISÉE SCELLÉE DANS UN RÉCIPIENT EN VERRE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **21.05.2018 JP 2018096902**

(43) Date of publication of application:
**31.03.2021 Bulletin 2021/13**

(73) Proprietor: **CHUGAI SEIYAKU KABUSHIKI KAISHA**
**Tokyo 115-8543 (JP)**

(72) Inventors:
• **YAMASHITA, Shogo**
**Tokyo 115-8543 (JP)**
• **YOSHIZAWA, Yuta**
**Tokyo 115-8543 (JP)**

(74) Representative: **Vossius & Partner**
**Patentanwälte Rechtsanwälte mbB**
**Siebertstrasse 3**
**81675 München (DE)**

(56) References cited:
WO-A1-2016/021510    JP-A- 2016 056 176
JP-A- 2017 141 307    JP-A- H0 948 639
JP-A- H08 325 037    JP-A- S63 146 826
US-A1- 2012 018 338

• ABDUL-FATTAH ET AL: "Investigating factors leading to fogging of glass vials in lyophilized drug products", EUROPEAN JOURNAL OF PHARMACEUTICS AND BIOPHARMACEUTICS,, vol. 85, 1 January 2013 (2013-01-01), pages 314 - 326, XP002785121

• "Schott Vials 40089 EN - Perfection in every detail - Schott AG", INTERNET CITATION, 1 September 2017 (2017-09-01), XP002785120, Retrieved from the Internet <URL:https://www.schott.com/d/.../schott-brochure-schott-vials-english-20092017.pdf> [retrieved on 20180926]

## Description

Technical Field

The present invention relates to a technique for suppressing fogging of glass containers for lyophilized formulations.

Background Art

Many pharmaceutical formulations use pharmaceutical compositions in liquid forms. Liquid formulations usually must be stored at low temperatures, and some pharmaceuticals in liquid forms degrade during storage. One possibility to overcome these problems is to lyophilize such a pharmaceutical composition. The pharmaceutical composition is transported and stored in a dry form and then reconstituted before use.

Particularly when the active agent is a protein, the lyophilization step itself may cause deterioration in the properties of the pharmaceutical composition. In order to prevent a decrease in the activity of the pharmaceutical composition, a surfactant is generally added to the pharmaceutical composition in addition to a cryoprotectant such as certain sugars.

A liquid composition containing a surfactant may adhere to a portion above the liquid level of a vial inner wall after filling. It is known that if the liquid composition is lyophilized as it is, the ingredients of the liquid composition adhere to and remain over a wide range of the vial inner wall, which may give the vial a "fogged" appearance (Patent Literature 1). Specifically, such "fogged" region on the inner surface of a glass vial suggests that a pre-lyophilization drug solution creeps up above the filling level followed by the drying of the pharmaceutical composition when the vial undergoes a lyophilization step, and then a white residue remains on the inner surface of the vial. Such residue is not only regarded as an apparent defect, but also it can affect the visual inspection of the vial or the quality inspection by automated equipment. Further, the bad appearance of the vial may be considered as a problem by a patient or a physician.

ABDUL-FATTAH ET AL ["Investigating factors leading to fogging of glass vials in lyophilized drug products",EUROPEAN JOURNAL OF PHARMACEUTICS AND BIOPHARMACEUTICS, vol. 85, 1 January 2013 (2013-01-01), pages 314-326] discloses a study explaining that a possible mechanism for film transfer to a glass solid surface is that in aqueous formulations, especially when containing a surfactant, a difference in the surface tension between two points on the solution surface (i.e., surface tension gradient) triggers a driving force and as a result a creeping behaviour is observed. D1 discloses that there is no systematic correlation between the fogging and the formulation properties. Hydrophilic surfaces, with lower contact angle, worsen the phenomenon of fogging. The study discloses that siliconized glass vials lead to no fogging due to the hydrophobicity of the glass surface.

Citation List

Patent Literature

Patent Literature 1: National Publication of International Patent Application No. 2012-520098

Summary of Invention

Technical Problem

The inventors have examined a method for suppressing the "fogging" on the inner surface of a glass container such as a vial caused by lyophilization, after the filling of the container with a pre-lyophilization drug solution, upon production of a lyophilized formulation of a pharmaceutical composition containing a surfactant. In the process, the inventors have found that a method for applying silicone to the inner surface of a glass container disclosed in National Publication of International Patent Application No. 2012-520098 is problematic in that the degree of shrinkage becomes stronger in cake formation during lyophilization, a gap is formed between the wall surface of the container and the cake, and the cake is easily moved. If the cake is easily moved, cake collapse may take place due to physical stress during transportation or the like, which may lead to quality deterioration. For example, if powder adheres to the vial inner wall due to the cake collapse and visibility deteriorates, quality inspection may be affected. Also, even if such cake collapse is considered only as an apparent defect, bad appearance of the vial can be considered as a problem by a patient or a physician and thus it is undesirable.

Therefore, the present invention provides a lyophilized formulation in which fogging of the inner surface of a glass container is prevented and furthermore good cake formation is achieved, a production method thereof, and the like.

Solution to Problem

As a result of intensive studies, the inventors have discovered that sulfur treatment or VIST treatment of the wall surface (inner surface) of a glass container for a lyophilized formulation suppresses the creeping up of a drug solution on the wall surface of the glass container upon lyophilization, prevents the "fogging" of the wall surface of the glass container after lyophilization, and allows good cake formation after lyophilization. The inventors have further studied and thus have completed the present invention.

Specifically, the present invention provides the following [1] to [11].

[1] A lyophilized formulation comprising a therapeutic agent and a surfactant sealed in a glass container, wherein an inner surface of the glass container is sulfur-treated or VIST-treated, and wherein the therapeutic agent is any one of a humanized anti-interleukin 6 (IL-6) receptor antibody, a Bi-specific humanized antibody of factor IX and factor X, and an anti-IL-31 receptor A humanized monoclonal antibody.

[2] The formulation according to [1], which is to be reconstituted in a solvent so that a concentration of the therapeutic agent in a reconstituted solution ranges from 0.01 to 300 mg/mL.

[3] The formulation according to [1] or [2], wherein the surfactant is a nonionic surfactant.

[4] The formulation according to [3], wherein the nonionic surfactant is a polyoxyethylene sorbitan fatty acid ester or a polyoxyethylene polyoxypropylene copolymer.

[5] The formulation according to [3] or [4] which is to be reconstituted in the solvent so that the concentration of the nonionic surfactant in the reconstituted solution ranges from 0.001 to 1% (w/v).

[6] The formulation according to any one of [1] to [5], which is to be reconstituted so that the reconstituted solution comprises: a therapeutic agent at 0.01 to 300 mg/mL; a buffer agent at 0 to 100 mM; a surfactant at 0.001 to 1% (w/v); and a stabilizing agent at 1 to 500 mM and/or a tonicity regulator at 5 to 500 mM.

[7] The formulation according to any one of [1] to [5], wherein a pre-lyophilization solution comprises: a therapeutic agent at 0.01 to 300 mg/mL; a buffer agent at 0 to 100 mM; a surfactant at 0.001 to 1% (w/v); and a stabilizing agent at 1 to 500 mM and/or a tonicity regulator at 5 to 500 mM.

[8] The formulation according to any one of [1] to [7], wherein the glass container is a vial or a prefilled syringe.

[9] A kit comprising the formulation according to any one of [1] to [8], wherein the kit further comprises a solvent for re-dissolution; or wherein the kit further comprises an instruction and/or a package insert for re-dissolution in a solvent.

[10] A method for producing a lyophilized formulation or a method for preventing or reducing fogging of a glass container for a lyophilized formulation, the method comprising (a) providing a glass container having a sulfur-treated or VIST-treated inner surface; (b) introducing a pre-lyophilization solution comprising a therapeutic agent and a surfactant into the glass container, wherein the therapeutic agent is any one of a humanized anti-interleukin 6 (IL-6) receptor antibody, a Bi-specific humanized antibody of factor IX and factor X, and an anti-IL-31 receptor A humanized monoclonal antibody; and (c) performing lyophilization.

[11] Use of a glass container having a sulfur-treated or VIST-treated inner surface in order to prevent or reduce fogging of glass upon lyophilization when the lyophilized formulation according to any one of [1] to [8] is produced.

Advantageous Effects of Invention

According to the present invention, the creeping up of a drug solution on the wall surface of a glass container upon lyophilization is suppressed to prevent fogging of the inner surface of the glass container after lyophilization and cake collapse, making it possible to provide a lyophilized formulation having high quality and excellent appearance.

Brief Description of Drawings

[Figure 1]Figure 1 shows that no fogging takes place on vials having a sulfur-treated or VIST-treated surfaces after lyophilization.

[Figure 2]Figure 2 shows that cake shrinkage is suppressed in vials having a sulfur-treated or VIST-treated surfaces.

[Figure 3]Figure 3 shows the fogging scores of lyophilized products containing various drug solutions sealed in untreated vials and sulfur-treated vials. Each sample name indicates [active pharmaceutical ingredient (API) ID]-[concentration]-[vial size].

[Figure 4]Figure 4 shows the fogging scores of lyophilized products containing antibody drug solutions sealed in untreated 10 mL vials and various surface-treated 10 mL vials. Each sample name indicates [active pharmaceutical ingredient (API) ID]-[concentration]-[vial size].

Description of Embodiments

The present invention provides a lyophilized formulation comprising a therapeutic agent and a surfactant sealed in a glass container, wherein an inner surface of the glass container is sulfur-treated or VIST-treated, and wherein the therapeutic agent is any one of a humanized anti-interleukin 6 (IL-6) receptor antibody, a Bi-specific humanized antibody of factor IX and factor X, and an anti-IL-31 receptor A humanized monoclonal antibody. In the lyophilized formulation of the present invention, fogging of the inner surface of a glass container is suppressed by suppressing the creeping up of a drug solution on the wall surface of the glass container upon lyophilization, and good cake formation is achieved.

In the present invention, the term "fogging" of the inner surface of a glass container refers to a state in which a residue of a pharmaceutical composition in a drug solution adheres to a level above the top of a cake contact point of the lyophilized formulation on of the inner wall of the glass container. The adhesion of a residue can usually be confirmed visually. It is considered that the fogging of the inner surface of a glass container takes place because a pharmaceutical composition in a liquid form introduced into the glass container creeps up the inner wall and is then subjected to lyophilization in that state. The expression "fogging is suppressed" means that the fogging score is lowered as compared with a case where a glass container before sulfur treatment or before VIST treatment is used. The fogging score is calculated by multiplying the evaluation scores of two parameters (evaluation items), that is, the fogging area (Area) by the fogging height (Height).

$$\text{Fogging score} = \text{Fogging area score (1)} \times \text{Fogging height score (2)}$$

(1) The fogging area (Area) refers to the area of a portion, to which a residue of a pharmaceutical composition in a drug solution adheres, on the wall surface from the top of a lyophilized cake contact point in a glass container to an upper end (usually a rubber plug stopper for a vial) where the residue of the pharmaceutical composition in the drug solution can adhere. The fogging area (%) is calculated with the area of the wall surface from the top of the lyophilized cake contact point to an upper end where the residue of the pharmaceutical composition in the drug solution can adhere, being 100%. Then the evaluation score is recorded according to the following criteria.

(a) The evaluation score of a fogging area is "0" when the fogging area (%) is 0%.
(b) The evaluation score of a fogging area is "1" when the fogging area (%) is greater than 0% and 5% or less.
(c) The evaluation score of a fogging area is "2" when the fogging area (%) is greater than 5% and 25% or less.
(d) The evaluation score of a fogging area is "3" when the fogging area (%) is greater than 25% and 50% or less.
(e) The evaluation score of a fogging area is "4" when the fogging area (%) is greater than 50% and 75% or less.
(f) The evaluation score of a fogging area is "5" when the fogging area (%) is greater than 75% and 100% or less.

(2) The fogging height (Height) refers to the height (distance) from the top of a lyophilized cake contact point on the inner wall of the glass container to the highest (longest) point where a residue of the pharmaceutical composition in the drug solution has adhered. The fogging height (%) is calculated with the height, from the top of the lyophilized cake contact point to an upper end (usually a rubber plug stopper for a vial) where the residue of the pharmaceutical composition in the drug solution can adhere, being 100%. Then the evaluation score is recorded according to the following criteria.

(a) The evaluation score of a fogging height is "0" when no fogging is observed.
(b) The evaluation score of a fogging height is "1" when the fogging height (%) is higher than 0% and 5% or less.
(c) The evaluation score of a fogging height is "2" when the fogging height (%) is higher than 5% and 25% or less.
(d) The evaluation score of a fogging height is "3" when the fogging height (%) is higher than 25% and 50% or less.
(e) The evaluation score of a fogging height is "4" when the fogging height (%) is higher than 50% and 75% or less.
(f) The evaluation score of a fogging height is "5" when the fogging height (%) is higher than 75% and 100% or less.

Further, when the fogging score is "less than 2", it can be determined that the fogging is not observed. In one embodiment, the lyophilized formulation of the present invention may have a fogging score of less than 2, less than 1.5, less than 1, less than 0.5, or 0.

In the present invention, the glass container is not particularly limited as long as it is made of a material and has a shape suitable for filling with a pharmaceutical composition in a liquid form and lyophilization. Examples of the material of the glass container include borosilicate glass or soda lime glass, and borosilicate glass is preferable. More specifically, examples thereof include, but are not limited to, materials conforming to the Japanese Pharmacopoeia (Pharmacopoeial tests, Tests for Containers and Packing Materials, Test for Glass Containers for Injections (3) Soluble alkali test (i) method 1), the European pharmacopoeia (3.2.1 GLASS CONTAINERS FOR PHARMACEUTICAL USE) and the US Pharmacopoeia (660. CONTAINERS). Moreover, examples of the shape of the glass container include vials, prefilled syringes, and cartridges. The prefilled syringe may be a double chamber prefilled syringe to facilitate the dissolution of the lyophilized formulation. The shape of the cartridge is not particularly limited. Examples thereof include a single chamber and a double chamber. It is more desirable if it has a shape suitable for self-injection.

In the present invention, the term "sulfur treatment" usually refers to chemical treatment for the purpose of suppressing an elution of alkali components from a glass, by which an elutable ingredient on the glass surface is reacted with a sulfur compound such as an aqueous ammonium sulfate solution at a high temperature so as to remove the ingredient as a water-soluble ingredient. For example, an aqueous ammonium sulfate solution is added to a container and then heated at 550°C to 650°C, so that the inner surface of the glass container can be subjected to sulfur treatment.

The sulfur-treated glass container in the present invention may be commercially available, and examples thereof include those provided by Murase Glass Co., Ltd., Shiotani Glass Co., Ltd. and the like.

In the present invention, the term "VIST treatment" refers to an alkali elution reduction treatment of a glass container developed by Daiwa Special Glass Co., Ltd., which is performed by washing the inner surface of a glass container with water, an aqueous solution of acid, an aqueous surfactant solution, or an aqueous solution of acid supplemented with a surfactant. Examples of acid that can be used herein include organic acids such as formic acid, acetic acid, oxalic acid, phthalic acid and citric acid, and inorganic acids such as hydrochloric acid, sulfuric acid and nitric acid, and citric acid is preferably used. The surfactant is not particularly limited, and for example, a nonionic surfactant can be used. VIST treatment can be performed by a known method, in which case WO2009/116300 can be referred to.

The VIST-treated glass container in the present invention may be a commercially available one, for example, a VIST-treated glass container provided by Daiwa Special Glass Co., Ltd.

As treatment for modifying a glass surface, siliconization by which dimethyl silicone is applied to a glass container and the glass container is baked at a high temperature is known. However, in a siliconized glass container, the shrinkage of a cake of the lyophilized formulation tends to take place, the cake is moved in the glass container, and the cake tends to be crushed during transportation or the like. On the other hand, in a sulfur-treated or VIST-treated glass container, such a problem does not occur because the shrinkage of a cake of the lyophilized formulation hardly takes place. In particular, a sulfur-treated glass container has a high effect of suppressing cake shrinkage. Therefore, in a preferred aspect of the present invention, a glass container having a sulfur-treated inner surface can be used. In addition, the sulfur treatment on a glass container has an advantage that it can be performed at a lower cost than that of siliconization.

Here, the term "cake" refers to a cake that is lyophilized after the filling of a glass container with a pre-lyophilization solution.

Here, the expression "cake shrinkage takes place" means that the cake is in a state where the cake is not fixed to the inner surface of a glass container. Such a state can be easily confirmed by visually observing whether or not the cake is moved in the glass container when the glass container is moved (for example, the glass container is inverted).

In the present invention, the surface treatment in a glass container may be applied to the entire inner surface or a part thereof. In a preferred aspect, a glass container having an entirely surface-treated inner surface can be used. In one aspect of the present invention, a glass container in which the surface treatment is applied to a region of the inner surface of the container above the top of a cake contact point after lyophilization can be used.

The lyophilized formulation of the present invention is produced by lyophilizing a pharmaceutical composition in a liquid form (specifically, a pre-lyophilization solution comprising a therapeutic agent and a surfactant) according to a method comprising:

(a) providing a glass container having a sulfur-treated or VIST-treated inner surface;
(b) introducing a pre-lyophilization solution comprising a therapeutic agent and a surfactant into the glass container, wherein the therapeutic agent is any one of a humanized anti-interleukin 6 (IL-6) receptor antibody, a Bi-specific humanized antibody of factor IX and factor X, and an anti-IL-31 receptor A humanized monoclonal antibody; and
(c) performing lyophilization.

Accordingly, a method for producing a lyophilized formulation, comprising the above steps, and a method for preventing or reducing the fogging of a glass container for the lyophilized formulation, comprising the above steps are also encompassed in the present invention. According to such a method of the present invention, the creeping up of a pre-lyophilization solution on the wall surface of the glass container is suppressed, thereby preventing or reducing the fogging of the inner surface of the glass container after lyophilization, and enabling good cake formation after lyophilization.

Lyophilization can be performed under conditions generally used in the pharmaceutical field. Lyophilization is usually carried out in three stages, namely freezing, primary drying and secondary drying.

In the freezing stage, a pharmaceutical composition in a liquid form is usually cooled to a temperature below the eutectic point. A pre-lyophilization solution is usually cooled to and frozen at -10°C to -80°C (for example, -20°C to -60°C) under atmospheric pressure.

In the primary drying stage, the pressure is lowered and the temperature is increased in order to sublimate a solvent. The temperature can range from -40°C to 50°C (for example, - 30°C to 40°C.). The pressure can range from 3 Pa to 80 Pa (for example, 5 Pa to 60 Pa). The primary drying stage is usually performed until at least about 90% of the solvent is removed.

In the secondary drying stage, an increased amount of the solvent is removed by increasing the temperature. The temperature can range from 10°C to 50°C (for example, 20°C to 40°C). The pressure can range from 3 Pa to 40 Pa (for example, 5 Pa to 30 Pa). When the secondary drying stage is complete, the moisture content of the lyophilized product is usually up to about 5%.

Before the freezing stage, a pre-cooling stage in which the temperature is lowered to 2°C to 10°C, for example, may be included.

The lyophilized formulation of the present invention contains at least one therapeutic agent. Any therapeutic agent that can be lyophilized can be applied to the present invention. Examples of the therapeutic agent also include a protein, a peptide, or a nucleic acid.

The concentration of the therapeutic agent in a pre-lyophilization solution depends on the type of the therapeutic agent, its use and the like. The concentration of the therapeutic agent is, for example, in the range of 0.01 to 300 mg/mL, in the range of 0.01 to 250 mg/mL, and in the range of 0.01 to 200 mg/mL.

The problem of the fogging of a container upon lyophilization tends to take place when the pre-lyophilization solution is a solution having a strong surface-active action. Or the problem of the fogging of a container upon lyophilization tends to take place when the pre-lyophilization solution is a solution containing a protein at a high concentration, etc. Thus, in a preferred aspect, the present invention is applied to protein solution formulations, particularly high concentration protein solution formulations for subcutaneous injection, etc. For example, the lyophilized formulation of the present invention can be obtained by placing a protein solution formulation in a sulfur-treated or VIST-treated glass container and then performing lyophilization.

In the present invention, the protein solution formulation is a solution formulation containing any one of a humanized anti-interleukin 6 (IL-6) receptor antibody, a Bi-specific humanized antibody of factor IX and factor X, and an anti-IL-31 receptor A humanized monoclonal antibody. The concentration of the protein in the protein solution formulation is, for example, 0.01 mg/mL or more, 1 mg/mL or more, 10 mg/mL or more, 50 mg/mL or more, 80 mg/mL or more, 100 mg/mL or more, 120 mg/mL or more, 150 mg/mL or more, less than 300 mg/mL, less than 250 mg/mL, or less than 200 mg/mL. In one aspect, the concentration of the protein in the pre-lyophilization solution in the present invention ranges from 0.01 mg/mL to 300 mg/mL, such as 1 to 300 mg/mL, 50 to 300 mg/mL, and the like.

In the present invention, the term "antibody-containing solution formulation" refers to formulation having an antibody concentration of 50 mg/mL or more, preferably 80 mg/mL or more, more preferably 100 mg/mL or more, further preferably 120 mg/mL or more, and even further preferably 150 mg/mL or more.

Further, the upper limit of the antibody concentration in the antibody-containing solution formulation is generally 300 mg/mL, preferably 250 mg/mL, and further preferably 200 mg/mL from the viewpoint of production. Therefore, the antibody concentration of the antibody solution ranges from preferably 50 to 300 mg/mL, more preferably 100 to 300 mg/mL, further preferably 120 to 250 mg/mL, and particularly preferably 150 to 200 mg/mL.

Examples of the monoclonal antibody to be used in the present disclosure, but not part of the invention, include not only monoclonal antibodies derived from animals such as humans, mice, rats, hamsters, rabbits, sheep, camels, and monkeys, but also artificially modified recombinant antibodies such as chimeric antibodies, humanized antibodies, and bispecific antibodies. Further examples thereof include recombinant antibodies prepared by artificially modifying variable regions and/or constant regions of the antibody etc., to modify the physical properties of an antibody molecule (specifically, isoelectric point (pI) modification, Fc receptor affinity modification, etc.) for the purpose of improving blood retention and pharmacokinetics.

Further, the immunoglobulin class of an antibody is not particularly limited, and may be any class including IgG such as IgG1, IgG2, IgG3 and IgG4, IgA, IgD, IgE, IgM, etc. IgG and IgM are preferred.

Furthermore, examples of an antibody in the present disclosure but not part of the invention include not only antibodies having constant regions and variable regions (whole antibodies), but also antibody fragments such as Fv, Fab, F(ab)$_2$, and low molecular weight antibodies such as monovalent or at least bivalent single-chain Fv (scFv, sc(Fv)$_2$) and diabodies including scFv dimers, which are obtained by connecting variable regions of the antibody using linkers such as peptide linkers. Whole antibodies are preferred, however.

The above antibodies to be used in the present invention can be prepared by methods well known to those skilled in the art. A hybridoma producing a monoclonal antibody can be basically prepared using a known technique as follows. Specifically, a hybridoma can be prepared by immunizing with a desired antigen or a cell expressing a desired antigen as a sensitizing antigen according to a normal immunization method, fusing the resulting immune cell with a known parent cell by a normal cell fusion method, and then screening for monoclonal antibody-producing cells (hybridomas) by a normal screening method. A hybridoma can be prepared, for example, according to Milstein et al's method (Kohler. G. and Milstein, C., Methods Enzymol. (1981) 73: 3-46). When the immunogenicity of an antigen is low, an immunization may be performed by binding to an immunogenic macromolecule such as albumin.

Further, a recombinant antibody produced by cloning an antibody gene from a hybridoma, incorporating it into a suitable vector, introducing the vector into a host, and using a gene recombination technique can be used (for example, see Carl, A. K. Borrebaeck, James, W. Larrick, THERAPEUTIC MONOCLONAL ANTIBODIES, Published in the United Kingdom by MACMILLAN PUBLISHERS LTD, 1990). Specifically, cDNA of a variable region (V region) of the antibody is synthesized from hybridoma mRNA using reverse transcriptase. When DNA encoding a V region of the antibody of interest is obtained, the DNA is ligated to DNA encoding a desired constant region (C region) of the antibody and then the resultant is incorporated into an expression vector. Alternatively, DNA encoding an V region of the antibody may be incorporated into an expression vector containing the DNA of a C region of the antibody. Such DNA is incorporated into an expression vector for expression under the control of an expression control region such as an enhancer and a promoter. Next, host cells can be transformed with this expression vector to express the antibody.

In the present disclosure, an artificially modified recombinant antibody for the purpose of reducing the heteroantigenicity to humans, etc., such as a chimeric antibody or a humanized antibody, can be used. These modified antibodies can be produced using known methods. A chimeric antibody is an antibody composed of heavy chain and light chain variable regions of a non-human mammal, for example, a mouse antibody and heavy chain and light chain constant regions of a human antibody, and can be obtained by ligating a DNA encoding the variable region of the mouse antibody to a DNA encoding the constant region of the human antibody, incorporating the resultant into an expression vector, and then introducing the vector into a host for production.

A humanized antibody is also referred to as a reshaped human antibody, and is prepared by grafting an complementarity determining region (CDR) of a non-human mammal, for example, a mouse antibody into a complementarity determining region of a human antibody. General gene recombination techniques therefor are also known. Specifically, a humanized antibody is obtained by synthesizing a DNA sequence designed to ligate the CDR of a mouse antibody to the framework region (FR) of a human antibody by a PCR method from several oligonucleotides prepared to have an overlapping portion at the end, ligating the obtained DNA to DNA encoding a constant region of a human antibody, incorporating the resultant into an expression vector, and then introducing the vector into a host for production thereof (see European Patent Application Publication No. EP 239400, WO96/02576). As the FR of a human antibody to be ligated via CDR, one in which the complementarity determining region forms a favorable antigen binding site is selected. If necessary, an amino acid(s) in the framework region of a variable region of the antibody may be substituted so that the complementarity determining region of a reshaped human antibody forms an appropriate antigen binding site (Sato, K.et al., Cancer Res. (1993) 53, 851-856).

As a technique for substituting an amino acid(s) of an antibody in order to improve the activity, physical properties, pharmacokinetics, safety, etc. of the antibody, for example, techniques described below are also known. Examples of the antibody to be used in the present invention include antibodies subjected to such amino acid substitutions (including deletions and additions).

Techniques for amino acid substitution in a variable region of an IgG antibody, which have been reported, include humanization (Tsurushita N, Hinton PR, Kumar S., Design of humanized antibodies: from anti-Tac to Zenapax., Methods. 2005 May;36(1):69-83.), affinity maturation performed by amino acid substitution in a complementarity determining region (CDR) for the enhancement of binding activity (Rajpal A, Beyaz N, Haber L, Cappuccilli G, Yee H, Bhatt RR, Takeuchi T, Lerner RA, Crea R., A general method for greatly improving the affinity of antibodies by using combinatorial libraries., Proc Natl Acad Sci U.S.A. 2005 Jun 14;102(24):8466-71.), and the improvement of physicochemical stability by amino acid substitution of framework (FR) (Ewert S, Honegger A, Pluckthun A., Stability improvement of antibodies for extracellular and intracellular applications: CDR grafting to stable frameworks and structure-based framework engineering., Methods. 2004 Oct; 34(2): 184-99. Review). Further, as a technique for performing amino acid substitution in the Fc region of the IgG antibody, a technique for enhancing antibody-dependent cytotoxic activity (ADCC activity) and complement-dependent cytotoxic activity (CDC activity) is known (Kim SJ, Park Y, Hong HJ., Antibody engineering for the development of therapeutic antibodies., Mol Cells. 2005 Aug 31;20(1):17-29. Review.). Furthermore, a technique for amino acid substitution of Fc not only for enhancing such effector functions but also for improving the blood half-life of antibodies has been reported (Hinton PR, Xiong JM, Johlfs MG, Tang MT, Keller S, Tsurushita N., An engineered human IgG1 antibody with longer serum half-life., J Immunol. 2006 Jan 1;176(1):346-56., Ghetie V, Popov S, Borvak J, Radu C, Matesoi D, Medesan C, Ober RJ, Ward ES., Increasing the serum persistence of an IgG fragment by random mutagenesis., Nat Biotechnol. 1997 Jul;15(7):637-40.). Furthermore, various amino acid substitution techniques for constant regions for the purpose of improving physical properties of antibodies are also known (WO09/41613).

Further, a method for obtaining a human antibody is also known. For example, human lymphocytes are sensitized with a desired antigen or cells expressing the desired antigen in vitro, and the sensitized lymphocytes are fused with human myeloma cells, such as U266, so that a desired human antibody having binding activity to the antigen can also be obtained (see Japanese Patent Publication No. 1-59878). Further, a desired human antibody can be obtained by immunizing a transgenic animal having all repertoires of human antibody genes with an antigen (WO93/12227, WO92/03918, WO94/02602, WO94/25585, WO96/34096, WO96/33735). Furthermore, a technique for obtaining a human antibody by panning using a human antibody library is also known. For example, a variable region of a human antibody is expressed as a single chain antibody (scFv) on the phage surface by the phage display method, and a phage that binds to an antigen can be selected. By analyzing the gene of the selected phage, the DNA sequence encoding the variable region of the human antibody that binds to the antigen can be determined. If the DNA sequence of scFv that binds to an antigen is clarified, a suitable expression vector containing the sequence can be prepared and a human antibody can be obtained. These methods are already well known, and WO92/01047, WO92/20791, WO93/06213, WO93/11236, WO93/19172, WO95/01438, and WO95/15388 can be referred to. Such human antibodies are also included in the antibodies to be used in the present invention.

When an antibody gene is once isolated and introduced into an appropriate host to prepare an antibody, a combination of an appropriate host and an expression vector can be used. When eukaryotic cells are used as hosts, animal cells, plant cells, and fungal cells can be used. Known animal cells include (1) mammalian cells such as CHO, COS, myeloma, BHK (baby hamster kidney), HeLa, Vero, (2) amphibian cells, such as Xenopus oocytes, or (3) insect cells, such as sf9, sf21, and Tn5. Known plant cells include cells derived from the genus Nicotiana, such as cells derived from Nicotiana tabacum, and these cells may be subjected to callus culture. Known fungal cells include yeasts such as those of the genus Saccharomyces, e.g., Saccharomyces cerevisiae, and filamentous fungi such as those of the genus Aspergillus, e.g., Aspergillus niger. When prokaryotic cells are used, there are production systems using bacterial cells. Known bacterial cells include E. coli and Bacillus subtilis. An antibody can be obtained by introducing an antibody gene of interest into these cells by transformation, and then culturing the transformed cells in vitro.

Furthermore, the antibodies to be used in the present disclosure include modified products of antibodies. For example, antibodies conjugated to various molecules such as polyethylene glycol (PEG) and cytotoxic drugs can also be used (Farmaco. 1999 Aug 30;54(8):497-516., Cancer J. 2008 May-Jun;14(3):154-69.). The antibodies to be used in the present disclosure also include these modified products of antibodies. Such a modified product of an antibody can be obtained by chemically modifying an antibody. These methods have already been established in this field.

Examples of the antibody to be used in the present invention are any one of a humanized anti-interleukin 6 (IL-6) receptor antibody, a Bi-specific humanized antibody of factor IX and factor X, and an anti-IL-31 receptor A humanized monoclonal antibody.

Examples of a preferred reshaped humanized antibody include a humanized anti-interleukin 6 (IL-6) receptor antibody (tocilizumab, hPM-1 or MRA, see WO92/19759), a humanized anti-HM1.24 monoclonal antibody (see WO98/14580), a humanized anti-parathyroid hormone related peptide antibody (anti-PTHrP antibody) (see WO98/13388), a humanized anti-tissue factor antibody (see WO99/51743), an anti-glypican-3 humanized IgG1κ antibody (codrituzumab, GC33, see WO2006/006693), an anti-NR10 humanized antibody (see WO2009/072604), a Bi-specific humanized antibody of factor IX and factor X (ACE910, see WO2012/067176), and Nemolizumab (CIM331) antibody, that is an anti-IL-31 receptor A humanized monoclonal antibody. Particularly preferred as humanized antibodies are a humanized anti-IL-6 receptor antibody, an anti-NR10 humanized antibody, a Bi-specific humanized antibody of factor IX and factor X, and Nemolizumab (CIM331) antibody, that is, an anti-IL-31 receptor A humanized monoclonal antibody.

The human IgM antibody is preferably an anti-ganglioside GM3 recombinant human IgM antibody (see WO05/05636) and the like.

As the low molecular weight antibody, an anti-TPO receptor agonist Diabody (see WO02/33072), an anti-CD47 agonist Diabody (see WO01/66737) and the like are preferable.

In the present invention, the term "antibody having a low isoelectric point (low pI antibody)" refers to an antibody having a low isoelectric point, which is hardly present, particularly, in nature. Examples of the isoelectric point of such an antibody include, but are not limited to, 3.0 to 8.0, preferably 5.0 to 7.5, more preferably 5.0 to 7.0, and particularly preferably 5.0 to 6.5. Note that natural (or normal) antibodies are usually considered to have an isoelectric point in the range of 7.5 to 9.5.

Furthermore, the antibody is preferably a pi-modified antibody in which the pI of the antibody is lowered by modifying amino acid residues exposed on the surface of the antibody. Such a pi-modified antibody refers to an antibody having a pI lowered by 1 or more, preferably 2 or more, and more preferably 3 or more, than the pI of the antibody before modification. Examples of such a pI-modified antibody include, but are not limited to, SA237 (MAb1, H chain/SEQ ID NO: 1, L chain/SEQ ID NO: 2), which is an anti-IL-6 receptor antibody described in WO2009/041621, an anti-NR10 humanized antibody, and a fully humanized NS22 antibody prepared by the method described in Example 12 of WO2009/072604.

Examples of amino acid residues exposed on the surface of an antibody include, but are not limited to, in the case of the H chain variable region, those selected from among amino acid residues based on Kabat numbering, H1, H3, H5, H8, H10, H12, H13, H15, H16, H19, H23, H25, H26, H31, H39, H42, H43, H44, H46, H61, H62, H64, H65, H68, H71, H72, H73, H75, H76, H81, H82b, H83, H85, H86, H105, H108, H110, and H112. Examples of the same include, but are not limited to, in the case of the L chain variable region, those selected from among amino acid residues based on Kabat numbering, L1, L3, L7, L8, L9, L11, L12, L16, L17, L18, L20, L22, L24, L27, L38, L39, L41, L42, L43, L45, L46, L49, L53, L54, L55, L57, L60, L63, L65, L66, L68, L69, L70, L74, L76, L77, L79, L80, L81, L85, L100, L103, L105, L106, and L107.

In the present invention, the term "modification" refers to the substitution of an original amino acid residue(s) with another amino acid residue(s), the deletion of the original amino acid residue(s), the addition of a new amino acid residue(s), etc. Preferably, the term refers to the substitution of the original amino acid residue(s) with another amino acid residue(s).

It is known that there are charged amino acids among amino acids. Generally, lysine (K), arginine (R), and histidine (H) are known as positively charged amino acids (positively charged amino acids). As negatively charged amino acids (negatively charged amino acids), aspartic acid (D), glutamic acid (E) and the like are known. The other amino acids are known as non-charged amino acids.

Amino acid residues after modification in the present invention are preferably appropriately selected from amino acid residues included in either the following group (a) or (b), but are not particularly limited to these amino acids.

(a) glutamic acid (E), aspartic acid (D)
(b) lysine (K), arginine (R), histidine (H)

In addition, when an unmodified amino acid residue is charged in advance, one preferred aspect is to modify the charged one into a non-charged amino acid residue.

Specifically, examples of modification in the present invention include (1) the substitution of a charged amino acid with a non-charged amino acid, (2) the substitution of a charged amino acid with an oppositely charged amino acid, and (3) the substitution of a non-charged amino acid with a charged amino acid.

The value of an isoelectric point can be measured by isoelectric focusing known to those skilled in the art. The value of a theoretical isoelectric point can be calculated using gene and amino acid sequence analysis software (Genetyx etc.).

An antibody having an amino acid residue with a modified charge can be obtained by modifying a nucleic acid encoding an antibody, culturing the nucleic acid in host cells, and purifying the antibody from the host cell culture. In the present invention, the expression "modifying a nucleic acid" refers to modifying a nucleic acid sequence to have a codon corresponding to an amino acid residue to be introduced by modification. More specifically, the expression refers to modifying the nucleotide sequence of a nucleic acid so that the codon of an amino acid residue before modification is modified to be the codon of an amino acid residue to be introduced by modification. That is, the codon encoding an amino acid residue to be modified is replaced by the codon encoding an amino acid residue to be introduced by modification. Such nucleic acid modification can be appropriately performed by those skilled in the art using known techniques such as site-directed mutagenesis and PCR mutagenesis.

The protein also disclosed in the present description may be a physiologically active protein that can be used as a pharmaceutical other than an antibody. Examples of such physiologically active proteins include, but are not limited to, a granulocyte colony-stimulating factor (G-CSF), a granulocyte-macrophage colony-stimulating factor (GM-CSF), hematopoietic growth factors such as erythropoietin (EPO) and thrombopoietin, interferon, cytokines such as IL-1 and IL-6, monoclonal antibodies, tissue plasminogen activator (TPA), urokinase, serum albumin, blood coagulation factor VIII, leptin, insulin, stem cell growth factor (SCF).

The physiologically active protein has substantially the same biological activity as that of a mammal, particularly a human physiologically active protein, and examples thereof include those derived from natural sources, preferably those obtained by gene recombination methods. As the physiologically active protein obtained by a gene recombination method, those produced by allowing bacteria such as Escherichia coli; yeast, animal-derived cultured cells such as Chinese hamster ovary (CHO) cells, C127 cells, and COS cells to produce the protein, and extracting and separating and purifying the resultant by various methods. Proteins obtained by gene recombination methods include those having the same amino acid sequence as that of a natural protein, or those having one or more deletions, substitutions, or addition in the amino acid sequence and having the biological activity. Furthermore, examples of the physiologically active protein also include those chemically modified with PEG or the like.

Examples of the physiologically active protein include proteins having sugar chains. The origin of a sugar chain is not particularly limited, but a sugar chain to be added to mammalian cells is preferable. Examples of mammalian cells include CHO cells, BHK cells, COS cells, human-derived cells, etc. Among them, CHO cells are most preferable.

For example, when the physiologically active protein is G-CSF, any G-CSF purified to a high purity can be used. The G-CSF in the present invention may be those produced by any method. G-CSF to be used herein is those produced by culturing a human tumor cell line, extracting, separating and purifying the resultant by various methods, or, those obtained by producing G-CSF in bacteria such as Escherichia coli, yeast, animal-derived cultured cells such as Chinese hamster ovary (CHO) cells, C127 cells, COS cells, etc., by genetic engineering techniques, and then extracting, separating and purifying the resultant by various methods. Preferably, G-CSF is produced by a gene recombination method using E. coli, yeast or CHO cells. Most preferred are those produced by CHO cells using a gene recombination method. Furthermore, G-CSF chemically modified with PEG or the like is also included (see International Patent Application Publication No. WO90/12874).

The buffer to be used in the protein-containing solution formulation is prepared using a buffer agent that is a substance for maintaining the pH of a solution. For the antibody-containing solution formulation, the pH of the solution ranges from preferably 4 to 8, more preferably 5.0 to 7.5, still more preferably 5.5 to 7.2, and even more preferably 6.0 to 6.5. The buffer agent that can be used in the present invention can adjust the pH in these ranges and is pharmaceutically acceptable. Such buffer agents are known to those skilled in the art of solution formulation and for example, inorganic salts such as phosphate (sodium or potassium), sodium bicarbonate; organic acid salts such as citrate (sodium or potassium), sodium acetate, and sodium succinate; or acids such as phosphoric acid, carbonic acid, citric acid, succinic acid, malic acid, and gluconic acid can be used. Furthermore, Tris and Good buffer agents such as MES, MOPS, and HEPES, histidine (for example, histidine hydrochloride), glycine, and the like may be used.

In the antibody-containing solution formulation, the buffer is preferably a histidine buffer or a glycine buffer, and particularly preferably a histidine buffer. The concentration of the buffer ranges from generally 1 to 500 mM, preferably 5 to 100 mM, and more preferably 10 to 20 mM. When a histidine buffer is used, the buffer preferably contains 5 to 25 mM histidine, and more preferably 10 to 20 mM histidine.

The antibody-containing solution formulation is preferably stabilized by adding a stabilizing agent suitable for an antibody as an active ingredient. A "stable" antibody-containing solution formulation is observed to have no significant change at refrigeration temperatures (2°C to 8°C) for at least 12 months, preferably 2 years, and more preferably 3 years; or at room temperature (22°C to 28°C) for at least 3 months, preferably 6 months, and more preferably for one year. For example, the total amount of dimers and degradation products after storage at 5°C for 2 years accounts for 5.0% or less, preferably 2% or less, and more preferably 1.5% or less, or the same after storage at 25°C for 6 months accounts for 5.0% or less, preferably 2% or less, and more preferably 1.5% or less.

The lyophilized formulation of the present invention contains at least one kind of surfactant.

Typical examples of the surfactant can include: nonionic surfactants such as sorbitan fatty acid esters such as sorbitan monocaprylate, sorbitan monolaurate, and sorbitan monopalmitate; glycerin fatty acid esters such as glycerin monocaprylate, glycerin monomyristate, and glycerin monostearate; polyglycerin fatty acid esters such as decaglyceryl monostearate, decaglyceryl distearate, and decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, and polyoxyethylene sorbitan tristearate; polyoxyethylene sorbit fatty acid esters such as polyoxyethylene sorbit tetrastearate and polyoxyethylene sorbit tetraoleate; polyoxyethylene glycerin fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol ether, polyoxyethylene polyoxypropylene propyl ether, and polyoxyethylene polyoxypropylene cetyl ether; polyoxyethylene alkyl phenyl ethers such as polyoxyethylene nonylphenyl ether; polyoxyethylene hydrogenated castor oils such as polyoxyethylene castor oil, and polyoxyethylene hydrogenated castor oil (polyoxyethylene hydrogen castor oil); polyoxyethylene beeswax derivatives such as polyoxyethylene sorbit beeswax; polyoxyethylene lanolin derivatives such as polyoxyethylene lanolin; those having HLB 6 to 18 such as polyoxyethylene fatty acid amides e.g., polyoxyethylene stearamide; anionic surfactants such as an alkyl sulfate salts having an alkyl group having 10 to 18 carbon atoms, such as sodium cetyl sulfate, sodium lauryl sulfate and sodium oleyl sulfate; polyoxyethylene alkyl ether sulfates wherein the average molar number of ethylene oxide ranges from 2 to 4 and the number of carbon atoms of an alkyl group ranges from 10 to 18, such as sodium polyoxyethylene lauryl sulfate; alkyl sulfosuccinate ester salts having an alkyl group having 8 to 18 carbon atoms, such as sodium lauryl sulfosuccinate; natural surfactants such as lecithin, and glycerophospholipid; fingophospholipids such as sphingomyelin; and sucrose fatty acid esters of fatty acids having 12 to 19 carbon atoms. The lyophilized formulation of the present invention can contain one or a combination of two or more kinds of these surfactants.

Preferred surfactants are nonionic surfactants, such as polyoxyethylene sorbitan fatty acid esters and polyoxyethylene polyoxypropylene copolymers, and particularly preferred are polysorbates 20, 21, 40, 60, 65, 80, 81, 85 and pluronic surfactants, and most preferred are polysorbates 20, 80 and pluronic F-68 (poloxamer 188).

The amount of a surfactant to be added to the pre-lyophilization solution ranges from generally 0.0001 to 10% (w/v). In one aspect, the concentration of a nonionic surfactant in the pre-lyophilization solution in the present invention ranges from 0.001 to 1% (w/v), such as 0.001 to 5% (w/v), and 0. 005 to 3% (w/v).

The lyophilized formulation of the present invention can appropriately contain pharmaceutically acceptable ingredients as necessary. Examples thereof include suspending agents, solubilizers, preservatives, adsorption inhibitors, diluents, excipients, pH adjusters, soothing agents, sulfur-containing reducing agents, and antioxidants.

Examples of the suspending agents can include methyl cellulose, polysorbate 80, hydroxyethyl cellulose, gum arabic, tragacanth powder, sodium carboxymethyl cellulose, and polyoxyethylene sorbitan monolaurate.

Examples of the solubilizers can include polyoxyethylene hydrogenated castor oil, polysorbate 80, nicotinic acid amide, polyoxyethylene sorbitan monolaurate, Magrogol, and castor oil fatty acid ethyl ester.

Examples of the isotonic agents can include sodium chloride, potassium chloride, and calcium chloride.

Examples of the preservatives can include methyl paraoxybenzoate, ethyl paraoxybenzoate, sorbic acid, phenol, cresol, and chlorocresol.

Examples of the adsorption inhibitors can include human serum albumin, lecithin, dextran, ethylene oxide-propylene oxide copolymer, hydroxypropyl cellulose, methyl cellulose, polyoxyethylene hydrogenated castor oil, and polyethylene glycol.

Examples of the sulfur-containing reducing agents include N-acetylcysteine, N-acetylhomocysteine, thioctic acid, thiodiglycol, thioethanolamine, thioglycerol, thiosorbitol, thioglycolic acid and a salt thereof, sodium thiosulfate, glutathione, and those having a sulfhydryl group such thioalkanoic acid having 1 to 7 carbon atoms.

Examples of the antioxidants include erythorbic acid, dibutylhydroxytoluene, butylhydroxyanisole, α-tocopherol, tocopherol acetate, L-ascorbic acid and a salt thereof, L-ascorbic acid palmitate, L-ascorbic acid stearate, sodium hydrogen sulfite, sodium sulfite, triamyl gallate, propyl gallate or chelating agents such as disodium ethylenediaminetetraacetate (EDTA), sodium pyrophosphate and sodium metaphosphate.

In addition, the lyophilized formulation of the present invention can appropriately contain a stabilizing agent and/or a tonicity regulator as necessary.

As used herein, the term "stabilizing agent" refers to a pharmaceutically acceptable additive that protects a therapeutic agent and/or formulation from chemical and/or physical degradation during manufacturing, storage, and application. The chemical and physical degradation pathways of pharmaceuticals are as summarized in Cleland et al. (1993), Crit. Rev. Ther. Drug Carrier Syst. 10(4):307-77, Wang (1999) Int. J. Pharm. 185(2):129-88, Wang (2000) Int. J. Pharm. 203(1-2):1-60, and Chi et al. (2003) Pharm. Res. 20(9):1325-36. Examples of the stabilizing agent include sugars, amino acids, polyols, cyclodextrins (for example, hydroxypropyl-β-cyclodextrin, sulfobutylethyl-β-cyclodextrin, and β-cyclodextrin), polyethylene glycol (for example, PEG 3000, PEG 3350, PEG 4000 and PEG 6000), albumin (for example, human serum albumin (HSA), and bovine serum albumin (BSA)), salts (for example, sodium chloride, magnesium chloride, and calcium chloride), and chelating agents (for example, EDTA). The stabilizing agent may be present in a pre-lyophilization solution or a reconstituted solution in an amount of about 1 to about 500 mM, preferably in an amount of about 10 to about 300 mM, and more preferably in an amount of about 100 to about 300 mM.

As used herein, the term "tonicity regulator" refers to a pharmaceutically acceptable additive that is used for regulating the tonicity (osmotic oroperty) of a pre-lyophilization solution or a reconstituted solution. Examples of the tonicity regulator include sodium chloride, potassium chloride, glycerin, and any ingredient from the group of amino acids or sugars. The tonicity regulator may be present in a pre-lyophilization solution or a reconstituted solution in an amount of about 5 to about 500 mM, preferably in an amount of about 50 to about 300 mM.

In a preferred aspect, the pre-lyophilization solution in the present invention contains:

a therapeutic agent at about 0.01 to about 200 mg/mL;
a buffer agent at 0 to about 100 mM;
a surfactant at about 0.001 to about 1% (w/v); and
a stabilizing agent at about 1 to about 500 mM and/or a tonicity regulator at about 5 to about 500 mM.

In a preferred aspect, when the therapeutic agent is a protein, the pre-lyophilization solution in the present invention contains:

the protein at about 50 to about 300 mg/mL;
a buffer agent at 0 to about 100 mM;
a surfactant at about 0.001 to about 1% (w/v); and
a stabilizing agent at about 1 to about 500 mM and/or a tonicity regulator at about 5 to about 500 mM.

The lyophilized formulation of the present invention is dissolved in a pharmaceutically acceptable solvent such as water for injection before use and administered to a subject as a reconstituted solution. The composition of the reconstituted solution may be the same as or different from the composition of the pre-lyophilization solution.

The concentration of the therapeutic agent in the reconstituted solution depends on the kind of the therapeutic agent, its use and the like. The concentration of the therapeutic agent is, for example, in the range of 0.01 to 300 mg/mL, in the range of 0.01 to 250 mg/mL, or in the range of 0.01 to 200 mg/mL.

In one aspect, when the therapeutic agent in the present invention is a protein, the lyophilized formulation of the present invention is reconstituted in a solvent so that the concentration of the protein in the reconstituted solution is, for example, 0.01 mg or more, 1 mg/mL or more, 10 mg/mL or more, 50 mg/mL or more, 80 mg/mL or more, 100 mg/mL or more, 120 mg/mL or more, 150 mg/mL or more, less than 300 mg/mL, less than 250 mg/mL, or less than 200 mg/mL. In one aspect, the lyophilized formulation of the present invention is reconstituted in a solvent so that the concentration of the protein in the reconstituted solution ranges from 0.01 mg/mL to 300 mg/mL, such as 1 to 300 mg/mL, and 50 to 300 mg/mL.

In one aspect, the lyophilized formulation of the present invention is re-suspended in a solvent, so that the concentration of a nonionic surfactant in the reconstituted solution ranges from 0.001 to 1% (w/v), such as 0.001 to 5% (w/v), or 0.005 to 3% (w/v).

In a preferred aspect, the lyophilized formulation of the present invention is reconstituted so that the reconstituted solution contains:

a therapeutic agent at about 0.01 to about 200 mg/mL;
a buffer agent at 0 to about 100 mM;
a surfactant at about 0.001 to about 1% (w/v); and
a stabilizing agent at about 1 to about 500 mM and/or a tonicity regulator at about 5 to about 500 mM.

In a preferred aspect, when the therapeutic agent is a protein, the lyophilized formulation of the present invention is reconstituted so that the reconstituted solution contains:

the protein at about 50 to about 300 mg/mL;
a buffer agent at 0 to about 100 mM;
a surfactant at about 0.001 to about 1% (w/v); and
a stabilizing agent at about 1 to about 500 mM and/or a tonicity regulator at about 5 to about 500 mM.

The osmotic pressure ratio of the reconstituted solution prepared from the lyophilized formulation of the present invention ranges from about 0.5 to 4, more preferably about 0.7 to 2, and is further preferably about 1.

The lyophilized formulation of the present invention is dissolved in a pharmaceutically acceptable solvent and then administered by subcutaneous injection, intravenous injection, intramuscular injection or the like. In the formulation for subcutaneous injection, the dose per administration is large (for example, about 80 to 200 mg when the therapeutic agent is an antibody), but the amount of such injection solution is limited. The lyophilized formulation of the present invention is particularly useful for subcutaneous injection because a reconstituted solution containing a high concentration therapeutic agent can be prepared.

When a vial is used in the present invention, the volume of the vial ranges from 2 mL to 100 mL, and 3 to 20 mL, for example.

In one aspect, the amount of the pre-lyophilization solution accounts for 10% to 90% and, for example, 20% to 80% of the vial volume. For example, when the volume of a vial is 10 mL, the amount of the pre-lyophilization solution ranges from 1 mL to 9 mL, and 2 to 8 mL, for example.

In one aspect, the amount of a drug solution after re-dissolution accounts for 10% to 90% and, for example, 20% to 80% of the vial volume. For example, when the volume of a vial is 10 mL, the amount of the drug solution after re-dissolution ranges from 1 mL to 9 mL, and 2 to 8 mL, for example.

The present invention will be described in more detail with reference to the following examples, but the scope of the present invention is not limited thereto.

Examples

Example 1 Anti-fogging effect for glass vial through sulfur treatment or VIST treatment

The degree of fogging of the inner surface of a glass container was evaluated when a protein-containing drug solution is placed in a sulfur-treated, VIST-treated, siliconized or untreated glass vial and then lyophilized.

Vials used herein are 10 mL vials, white bulk, (material: borosilicate glass; no treatment; manufactured by Murase Glass Co., Ltd.), 10 mL vials, white sulfur bulk, (material: borosilicate glass; sulfur-treated; manufactured by Murase Glass Co., Ltd.), 10 mL VIST vials (material: borosilicate glass; VIST-treated; manufactured by Daiwa Special Glass Co., Ltd.) and 10 mL TopLyo (registered trademark) vials (material: borosilicate glass; siliconized; manufactured by SCHOTT AG). Each vial was used after dry heat sterilization at 250°C for 120 minutes.

As the protein-containing drug solution, a solution of an antibody (CIM331) (30 mg/mL), Tris buffer (6 mmol/L), sucrose (75 mmol/L), arginine (45 mmol/L), and poloxamer 188 (0.15 mg/mL) was prepared. CIM331 is an anti-human IL-31RA antibody described in WO2010/064697 A1 and WO2016/167263 A1, and was prepared by a method known to those skilled in the art according to the description of the patent documents.

5 mL of the protein-containing drug solution was placed in a vial, frozen at -45°C using Triomaster (manufactured by Kyowa Vacuum Engineering Co., Ltd.), subjected to primary drying at temperatures near the collapse temperature under vacuum conditions, and then subjected to secondary drying at 30°C under vacuum conditions.

After lyophilization, the degree of fogging on the inner surface of the glass container was evaluated based on the fogging score. The fogging score was calculated by multiplying the evaluation scores of two parameters (evaluation items), that is, the fogging area (Area) and the fogging height (Height) (see Table 1 below).

$$\text{Fogging score} = \text{Fogging area score (1)} \times \text{Fogging height score (2)}$$

(1) The fogging area (Area) refers to the area of a portion, to which a residue of the pharmaceutical composition in the drug solution adhered, on the glass wall surface from the top of the lyophilized cake contact point to the rubber plug stopper. The fogging area (%) was calculated with the area of the glass wall surface from the top of the lyophilized cake contact point to the rubber plug stopper, being 100%. Then the evaluation score was recorded according to the following criteria.

(a) The evaluation score of a fogging area is "0" when the fogging area (%) is 0%.
(b) The evaluation score of a fogging area is "1" when the fogging area (%) is greater than 0% and 5% or less.
(c) The evaluation score of a fogging area is "2" when the fogging area (%) is greater than 5% and 25% or less.
(d) The evaluation score of a fogging area is "3" when the fogging area (%) is greater than 25% and 50% or less.
(e) The evaluation score of a fogging area is "4" when the fogging area (%) is greater than 50% and 75% or less.
(f) The evaluation score of a fogging area is "5" when the fogging area (%) is greater than 75% and 100% or less.

(2) The fogging height (Height) refers to the height (distance) from the top of the lyophilized cake contact point to the highest (longest) point where the residue of the pharmaceutical composition in the drug solution has adhered. The fogging height (%) was calculated with the height from the top of the lyophilized cake contact point to the rubber plug stopper, being 100%. Then the evaluation score was recorded according to the following criteria.

(a) The evaluation score of a fogging height is "0" when no fogging is observed.
(b) The evaluation score of a fogging height is "1" when the fogging height (%) is higher than 0% and 5% or less.
(c) The evaluation score of a fogging height is "2" when the fogging height (%) is higher than 5% and 25% or less.
(d) The evaluation score of a fogging height is "3" when the fogging height (%) is higher than 25% and 50% or less.
(e) The evaluation score of a fogging height is "4" when the fogging height (%) is higher than 50% and 75% or less.
(f) The evaluation score of a fogging height is "5" when the fogging height (%) is higher than 75% and 100% or less.

When the above fogging score (Fogging score) was "less than 2", it was determined that the fogging was not observed.

[Table 1]

| | 0 | 1 | 2 | 3 | 4 | 5 |
|---|---|---|---|---|---|---|
| Area | 0% | ~5% | ~25% | ~50% | ~75% | ~100% |
| Height | 0% | ~5% | ~25% | ~50% | ~75% | ~100% |
| | | | | | | |
| Fogging score = (Area) x (Height) | | | | | | |

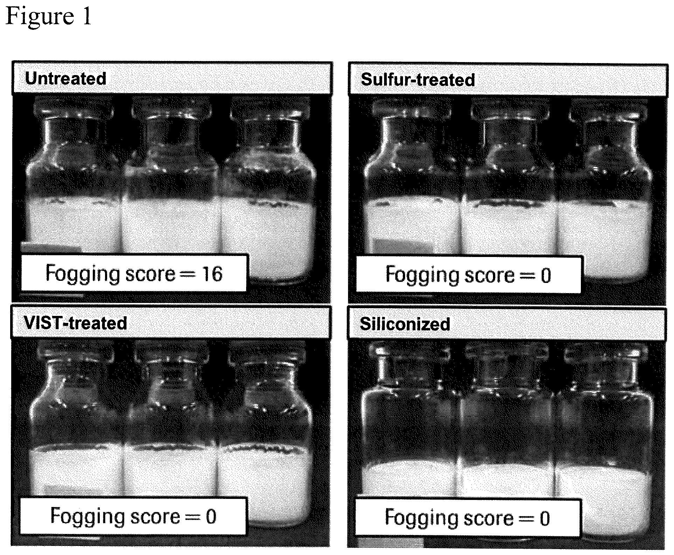

The results are shown in Fig 1. The sulfur-treated vials, the VIST-treated vials and the siliconized vials exhibited the fogging score of 0, indicating that no fogging was observed. On the other hand, the untreated vials exhibited the fogging score of 16, indicating that the fogging was observed. It is considered that the roughness of glass vial surfaces is alleviated through these treatments, so that wettability may be reduced to make the fogging difficult to take place.

Example 2 Sulfur-treated vials and VIST-treated vials cause no cake shrinkage

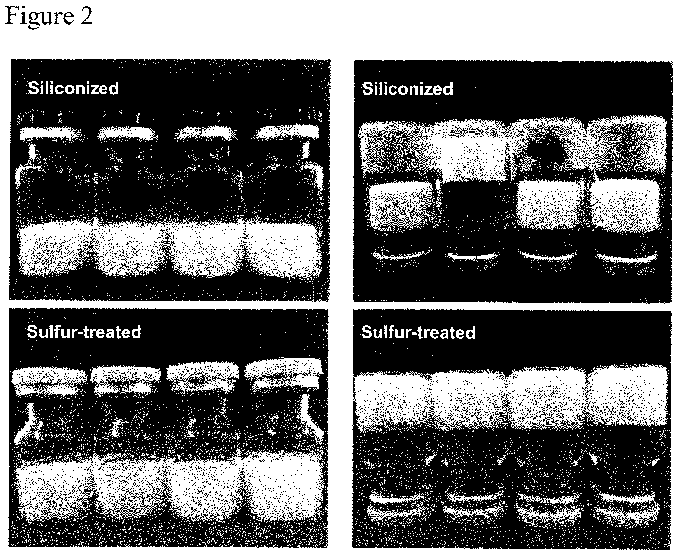

Vials subjected to various surface treatments were evaluated for the cake state after the preparation of lyophilized formulations in the same manner as in Example 1.
The results are shown in Figure 2. In siliconized vials, cake shrinkage took place upon lyophilization, and a gap was formed between the wall surface of the container and the cake, and the cake was found to be moved easily in the vial. On the other hand, in the sulfur-treated vials and the VIST-treated vials, no cake shrinkage took place and good cake formation was achieved (data not shown). If the cake is easily moved, the cake may collapse due to physical stress during transportation or the like, which may lead to quality deterioration. Thus, it was demonstrated that the sulfur-treated vials and the VIST-treated vials are superior to siliconized vials in that good cake formation is achieved in addition to the suppression of the fogging upon lyophilization.

Example 3 Evaluation of fogging score and cake moving (%) of lyophilized products containing various drug solutions sealed in various surface-treated vials

<Method>

Test samples: Protein solutions listed in Table 2 were prepared.

[Table 2]

| API ID | Formulation |
|---|---|
| mAb-A | **ACE910 30 mg/mL,** His 20 mM, Arg 150 mM, Asp 162 mM, Suc 150 mM, PX188 0.5 mg/mL |
| mAb-B | **MRA20 mg/mL,** Phosphate 14.3 mM, Suc 146 mM, PS80 0.5 mg/mL |
| mAb-C | **CIM331 15 mg/mL,** Tris/Tris-HCl 6 mM, Arg 45 mM, Suc 75 mM, PX188 0.15 mg/mL |
| Protein-250 | **G-CSF 0.25 mg/mL,** Arg 57 mM, Ph-Ala 61 mM, Met 7 mM, Man 137 mM, PS20 0.1 mg/mL |
| Protein-100 | **G-CSF 0.1 mg/mL,** Arg 57 mM, Ph-Ala 61 mM, Met 7 mM, Man 137 mM, PS20 0.1 mg/mL |
| Protein-50 | **G-CSF 0.05 mg/mL,** Arg 57 mM, Ph-Ala 61 mM, Met 7 mM, Man 137 mM, PS20 0.1 mg/mL |
| Placebo (incl. surfactant, excl. mAb) | **(Placebo)** Tris/Tris-HCl 6 mM, Arg 45 mM, Suc 75 mM, **PX188 0.15 mg/mL** |

Vial: As in Example 1, vials listed in Table 3 were used. However, for untreated vials (Normal) and sulfur-treated vials (Sulfur), 3 mL vials were also used. Each vial was used after dry heat sterilization at 250°C for 120 minutes.

[Table 3]

| | Size | Supplier |
|---|---|---|
| **Normal (Non-sulfur)** | 3 mL, 10 mL | Murase Glass |
| **Sulfur (Ammonium sulfate-treated)** | 3 mL, 10 mL | Murase Glass |
| **VIST (Citric acid-treated)** | 10 mL | Daiwa Special Glass |
| **TopLyo® (Siliconized)** | 10 mL | Schott AG |

Evaluation method: About 3 mL of a test sample was placed in a 10 mL vial, and about 1 mL of a test sample was placed in a 3 mL vial. After freezing at -45°C, primary drying was performed using Triomaster II-A04 (manufactured by Kyowa Vacuum Engineering Co., Ltd.) at a temperature near the collapse temperature under vacuum conditions, and secondary drying was performed at 30°C under vacuum conditions. Thereafter, the anti-fogging effect of a glass vial was evaluated in the same manner as in Example 1. Moreover, the cake moving (%) was calculated by observing cake moving when a vial was turned upside-down, and then cake shrinkage (desorption from the vial) was evaluated.

Cake moving (%) = Number of samples with cake moving / Number of observed samples x 100

<Result>

Anti-fogging effect of glass vial

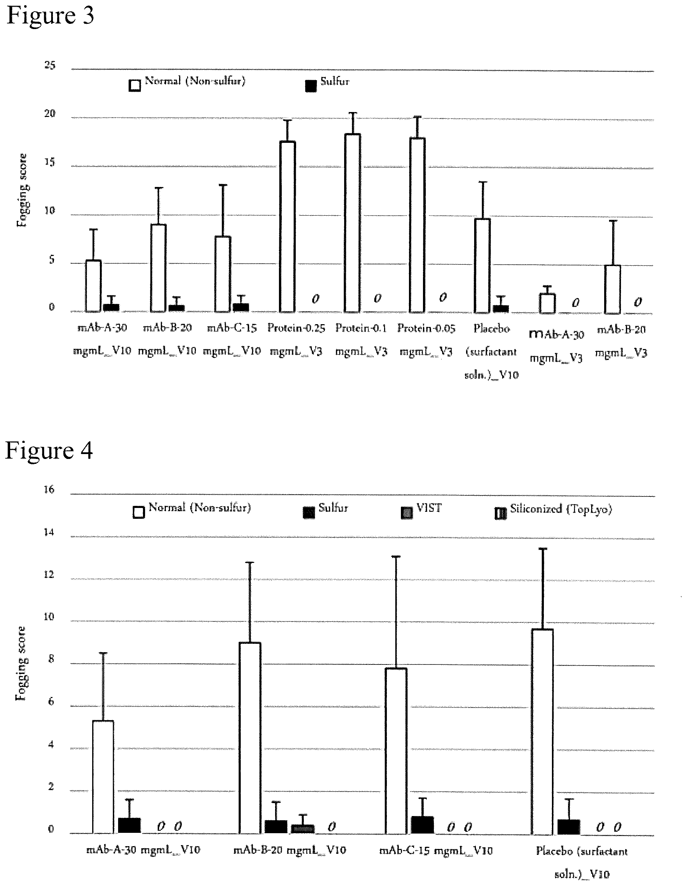

The results are shown in Table 4 and Figs. 3 and 4.

[Table 4]

| API | conc. | fill vol | Primary container | size | Fogging score Ave. | S.D. | N | Cakes moving with upside down — moved no | total | % |
|---|---|---|---|---|---|---|---|---|---|---|
| mAb-A | 30 mg/mL | 3 mL | Normal (Non-sulfur) | 10 mL | 5.3 | 3.2 | 9 | 0 | 13 | 0 |
| | | | Sulfur | | 0.7 | 0.9 | 13 | 0 | 8 | 0 |
| | | | VIST | | 0.0 | 0.0 | 8 | 7 | 7 | 100 |
| | | | Siliconized (TopLyo®) | | 0.0 | 0.0 | 7 | | | |
| mAb-B | 20 mg/mL | 3 mL | Non-sulfur | 10 mL | 9.0 | 3.8 | 9 | 6 | 13 | 46 |
| | | | Sulfur | | 0.6 | 0.9 | 13 | 7 | 8 | 88 |
| | | | VIST | | 0.4 | 0.5 | 8 | 7 | 7 | 100 |
| | | | Siliconized (TopLyo®) | | 0.0 | 0.0 | 7 | | | |
| mAb-C | 15 mg/mL | 3 mL | Non-sulfur | 10 mL | 7.8 | 5.3 | 6 | 4 | 16 | 25 |
| | | | Sulfur | | 0.8 | 0.9 | 16 | 4 | 6 | 67 |
| | | | VIST | | 0.0 | 0.0 | 6 | 6 | 6 | 100 |
| | | | Siliconized (TopLyo®) | | 0.0 | 0.0 | 6 | | | |
| Protein-250 | 0.25 mg/mL | 1 mL | Non-sulfur | 3 mL | 17.6 | 2.2 | 5 | | | |
| | | | Sulfur | | 0.0 | 0.0 | 5 | | | |
| Protein-100 | 0.1 mg/mL | 1 mL | Non-sulfur | 3 mL | 18.4 | 2.2 | 6 | | | |
| | | | Sulfur | | 0.0 | 0.0 | 3 | | | |
| Protein-50 | 0.05 mg/mL | 1 mL | Non-sulfur | 3 mL | 18.0 | 2.2 | 6 | | | |
| | | | Sulfur | | 0.0 | 0.0 | 4 | | | |
| Placebo (surfactant soln.) | 0 mg/mL | 3 mL | Non-sulfur | 10 mL | 9.7 | 3.8 | 6 | | | |
| | | | Sulfur | | 0.7 | 1.0 | 12 | | | |
| | | | VIST | | 0.0 | 0.0 | 5 | | | |
| mABb-A | 30 mg/mL | 1 mL | Non-sulfur | 3 mL | 2.0 | 0.8 | 4 | 4 | 12 | 33 |
| | | | Sulfur | | 0.0 | 0.0 | 5 | 2 | 5 | 40 |
| mAb-B | 20 mg/mL | 1 mL | Non-sulfur | 3 mL | 5.0 | 4.6 | 3 | | | |
| | | | Sulfur | | 0.0 | 0.0 | 4 | | | |

As shown in Table 4 and Figure 3, the sulfur-treated vials exhibited the fogging score of less than 2 for all test samples, indicating that no fogging was observed. On the other hand, the untreated vials exhibited the fogging score of 2 or more for all test samples, indicating that the fogging was observed.

Further, as shown in Table 4 and Figure 4, even in the VIST-treated vials and the siliconized vials, the lyophilized products in which various antibody solutions had been sealed exhibited the fogging score of less than 2, indicating that no fogging was observed.

Cake shrinkage (Table 4 and 5)

Results are shown in Table 4 and 5.

[Table 5]

| Table 5: Cake moving (%) when lyophilized products containing antibody drug solutions sealed in untreated 10 mL vials and various surface-treated 10 mL vials were turned upside-down. | | |
|---|---|---|
| API | Vial | Cake moving with upside-down |
| mAb-A | Sulfur | 0/13(0%) |
| | VIST | 0/8 (0%) |
| | Siliconized | 7/7 (100%) |
| mAb-B | Sulfur | 6/13 (46%) |
| | VIST | 7/8 (88%) |
| | Siliconized | 7/7 (100%) |
| mAb-C | Sulfur | 4/16 (25%) |
| | VIST | 4/6 (67%) |
| | Siliconized | 6/6 (100%) |
| Placebo (Surfactant soln.) | Sulfur | 4/12 (33%) |
| | VIST | 2/5 (40%) |

In the siliconized vials, cake moving took place in all vials for all test samples (cake moving (%): 100%). On the other hand, the VIST-treated vials exhibited lower cake moving (%) for all test samples compared to the siliconized vials, and the sulfur-treated vials exhibited even lower cake moving (%) than that of VIST-treated vials. Thus, the sulfur-treated vials and the VIST-treated vials were shown to cause less cake shrinkage than the siliconized vials.

Industrial Applicability

According to the present invention, in the lyophilized formulation, the fogging of the inner surface of a glass container and the cake collapse are prevented, so that a product having excellent appearance and quality can be provided. In addition, the fogging of the inner surface of a glass container is prevented, thereby reducing erroneous detection by an automatic inspection device, and realizing the more efficient quality control process for the lyophilized formulation.

**Claims**

1. A lyophilized formulation comprising a therapeutic agent and a surfactant sealed in a glass container, wherein an inner surface of the glass container is sulfur-treated or VIST-treated, and
wherein the therapeutic agent is any one of a humanized anti-interleukin 6 (IL-6) receptor antibody, a Bi-specific humanized antibody of factor IX and factor X, and an anti-IL-31 receptor A humanized monoclonal antibody.

2. The formulation according to claim 1, which is to be reconstituted in a solvent so that a concentration of the therapeutic agent in a reconstituted solution ranges from 0.01 to 300 mg/mL.

3. The formulation according to claim 1 or 2, wherein the surfactant is a nonionic surfactant.

4. The formulation according to claim 3, wherein the nonionic surfactant is a polyoxyethylene sorbitan fatty acid ester or a polyoxyethylene polyoxypropylene copolymer.

5. The formulation according to claim 3 or 4, which is to be reconstituted in the solvent so that the concentration of the nonionic surfactant in the reconstituted solution ranges from 0.001 to 1% (w/v).

**6.** The formulation according to any one of claims 1 to 5, which is to be reconstituted so that the reconstituted solution comprises:

    a therapeutic agent at 0.01 to 300 mg/mL;
    a buffer agent at 0 to 100 mM;
    a surfactant at 0.001 to 1% (w/v); and
    a stabilizing agent at 1 to 500 mM and/or a tonicity regulator at 5 to 500 mM.

**7.** The formulation according to any one of claims 1 to 5, wherein
a pre-lyophilization solution comprises:

    a therapeutic agent at 0.01 to 300 mg/mL;
    a buffer agent at 0 to 100 mM;
    a surfactant at 0.001 to 1% (w/v); and
    a stabilizing agent at 1 to 500 mM and/or a tonicity regulator at 5 to 500 mM.

**8.** The formulation according to any one of claims 1 to 7, wherein the glass container is a vial or a prefilled syringe.

**9.** A kit comprising the formulation according to any one of claims 1 to 8, wherein the kit further comprises a solvent for re-dissolution; or
wherein the kit further comprises an instruction and/or a package insert for re-dissolution in a solvent.

**10.** A method for producing a lyophilized formulation or a method for preventing or reducing fogging of a glass container for a lyophilized formulation, the method comprising

    (a) providing a glass container having a sulfur-treated or VIST-treated inner surface;
    (b) introducing a pre-lyophilization solution comprising a therapeutic agent and a surfactant into the glass container, wherein the therapeutic agent is any one of a humanized anti-interleukin 6 (IL-6) receptor antibody, a Bi-specific humanized antibody of factor IX and factor X, and an anti-IL-31 receptor A humanized monoclonal antibody; and
    (c) performing lyophilization.

**11.** Use of a glass container having a sulfur-treated or VIST-treated inner surface in order to prevent or reduce fogging of glass upon lyophilization when the lyophilized formulation according to any one of claims 1 to 8 is produced.

**Patentansprüche**

**1.** Eine lyophilisierte Formulierung, umfassend ein therapeutisches Mittel und ein grenzflächenaktives Mittel, verschlossen in einem Glasbehälter, wobei eine innere Oberfläche des Glasbehälters schwefelbehandelt oder VIST-behandelt ist und
wobei das therapeutische Mittel eines aus einem humanisierten Anti-Interleukin 6 (IL-6)-Rezeptor-Antikörper, einem Bi-spezifischen humanisierten Antikörper von Faktor IX und Faktor X und einem humanisierten monoklonalen Anti-IL-31-Rezeptor-A-Antikörper ist.

**2.** Die Formulierung nach Anspruch 1, die in einem Lösungsmittel rekonstituiert werden soll, so dass eine Konzentration des therapeutischen Mittels in einer rekonstituierten Lösung im Bereich von 0,01 bis 300 mg/ml liegt.

**3.** Die Formulierung nach Anspruch 1 oder 2, wobei das grenzflächenaktive Mittel ein nichtionisches grenzflächenaktives Mittel ist.

**4.** Die Formulierung nach Anspruch 3, wobei das nichtionische grenzflächenaktive Mittel ein Polyoxyethylen-Sorbitan-Fettsäureester oder ein Polyoxyethylen-Polyoxypropylen-Copolymer ist.

**5.** Die Formulierung nach Anspruch 3 oder 4, die in dem Lösungsmittel rekonstituiert werden soll, so dass die Konzentration des nichtionischen grenzflächenaktiven Mittels in der rekonstituierten Lösung im Bereich von 0,001 bis 1% (Gew./Vol.) liegt.

**6.** Die Formulierung nach einem der Ansprüche 1 bis 5, die so rekonstituiert werden soll, dass die rekonstituierte Lösung umfasst:

> ein therapeutisches Mittel von 0,01 bis 300 mg/ml;
> ein Puffermittel von 0 bis 100 mM;
> ein grenzflächenaktives Mittel von 0,001 bis 1% (Gew./Vol.); und
> ein Stabilisierungsmittel von 1 bis 500 mM und/oder einen Tonizitätsregler von 5 bis 500 mM.

**7.** Die Formulierung nach einem der Ansprüche 1 bis 5, wobei eine Vorlyophilisierungslösung umfasst:

> ein therapeutisches Mittel von 0,01 bis 300 mg/ml;
> ein Puffermittel von 0 bis 100 mM;
> ein grenzflächenaktives Mittel von 0,001 bis 1% (Gew./Vol.); und
> ein Stabilisierungsmittel von 1 bis 500 mM und/oder einen Tonizitätsregler von 5 bis 500 mM.

**8.** Die Formulierung nach einem der Ansprüche 1 bis 7, wobei der Glasbehälter ein Fläschchen oder eine vorgefüllte Spritze ist.

**9.** Ein Kit, umfassend die Formulierung nach einem der Ansprüche 1 bis 8, wobei das Kit außerdem ein Lösungsmittel zur Wiederauflösung enthält; oder wobei das Kit außerdem eine Anleitung und/oder eine Packungsbeilage für die Wiederauflösung in einem Lösungsmittel umfasst.

**10.** Ein Verfahren zur Herstellung einer lyophilisierten Formulierung oder ein Verfahren zur Verhinderung oder Verringerung des Beschlagens eines Glasbehälters für eine lyophilisierte Formulierung, wobei das Verfahren umfasst

> (a) Bereitstellen eines Glasbehälters mit einer schwefelbehandelten oder VIST-behandelten inneren Oberfläche;
> (b) Einbringen einer Vorlyophilisierungslösung, die ein therapeutisches Mittel und ein grenzflächenaktives Mittel umfasst, in den Glasbehälter, wobei das therapeutische Mittel eines aus einem humanisierten Anti-Interleukin 6 (IL-6)-Rezeptor-Antikörper, einem Bi-spezifischen humanisierten Antikörper von Faktor IX und Faktor X und einem humanisierten monoklonalen Anti-IL-31-Rezeptor-A-Antikörper ist; und
> (c) Durchführen einer Lyophilisierung.

**11.** Verwendung eines Glasbehälters mit einer schwefelbehandelten oder VIST-behandelten inneren Oberfläche, um das Beschlagen des Glases bei der Lyophilisierung zu verhindern oder zu verringern, wenn die lyophilisierte Formulierung nach einem der Ansprüche 1 bis 8 hergestellt wird.

**Revendications**

**1.** Formulation lyophilisée comprenant un agent thérapeutique et un tensioactif, scellée dans un récipient en verre, dans laquelle une surface intérieure du récipient en verre est traitée au soufre ou traitée au VIST, et dans laquelle l'agent thérapeutique est l'un quelconque parmi un anticorps anti-récepteur d'interleukine 6 (IL-6) humanisé, un anticorps humanisé bispécifique du facteur IX et du facteur X, et un anticorps monoclonal humanisé anti-récepteur A d'IL-31.

**2.** Formulation selon la revendication 1, qui est destinée à être reconstituée dans un solvant de façon qu'une concentration de l'agent thérapeutique dans une solution reconstituée soit située dans la plage allant de 0,01 à 300 mg/ml.

**3.** Formulation selon la revendication 1 ou 2, dans laquelle le tensioactif est un tensioactif non-ionique.

**4.** Formulation selon la revendication 3, dans laquelle le tensioactif non-ionique est un ester d'acide gras et de sorbitan polyoxyéthyléné ou un copolymère de poly(oxyde d'éthylène) et de poly(oxyde de propylène).

**5.** Formulation selon la revendication 3 ou 4, qui est destinée à être reconstituée dans le solvant de façon que la concentration du tensioactif non-ionique dans la solution reconstituée soit située dans la plage allant de 0,001 à 1 % (p/v).

**6.** Formulation selon l'une quelconque des revendications 1 à 5, qui est destinée à être reconstituée de façon que la solution reconstituée comprenne :

un agent thérapeutique à raison de 0,01 à 300 mg/ml ;
un agent tampon à raison de 0 à 100 mM ;
un tensioactif à raison de 0,001 à 1 % (p/v) ; et
un agent stabilisant à raison de 1 à 500 mM et/ou un régulateur de tonicité à raison de 5 à 500 mM.

**7.** Formulation selon l'une quelconque des revendications 1 à 5, dans laquelle une solution de pré-lyophilisation comprend :

un agent thérapeutique à raison de 0,01 à 300 mg/ml ;
un agent tampon à raison de 0 à 100 mM ;
un tensioactif à raison de 0,001 à 1 % (p/v) ; et
un agent stabilisant à raison de 1 à 500 mM et/ou un régulateur de tonicité à raison de 5 à 500 mM.

**8.** Formulation selon l'une quelconque des revendications 1 à 7, dans laquelle le récipient en verre est une ampoule ou une seringue préremplie.

**9.** Trousse comprenant la formulation selon l'une quelconque des revendications 1 à 8,

dans laquelle la trousse comprend en outre un solvant pour une re-dissolution ; ou
dans laquelle la trousse comprend en outre une instruction et/ou une notice pour une re-dissolution dans un solvant.

**10.** Méthode pour produire une formulation lyophilisée ou méthode pour empêcher ou réduire l'embuage d'un récipient en verre pour une formulation lyophilisée, la méthode comprenant

(a) la fourniture d'un récipient en verre ayant une surface intérieure traitée au soufre ou traitée au VIST ;
(b) l'introduction d'une solution de pré-lyophilisation comprenant un agent thérapeutique et un tensioactif dans le récipient en verre, dans laquelle l'agent thérapeutique est l'un quelconque parmi un anticorps anti-récepteur d'interleukine 6 (IL-6) humanisé, un anticorps humanisé bispécifique du facteur IX et du facteur X, et un anticorps monoclonal humanisé anti-récepteur A d'IL-31 ; et
(c) la mise en oeuvre d'une lyophilisation.

**11.** Utilisation d'un récipient en verre ayant une surface intérieure traitée au soufre ou traitée au VIST afin d'empêcher ou de réduire l'embuage du verre lors d'une lyophilisation quand la formulation lyophilisée selon l'une quelconque des revendications 1 à 8 est produite.

Figure 1

# Figure 2

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012520098 A
- WO 2009116300 A
- EP 239400 A
- WO 9602576 A
- WO 0941613 A
- JP 1059878 A
- WO 9312227 A
- WO 9203918 A
- WO 9402602 A
- WO 9425585 A
- WO 9634096 A
- WO 9633735 A
- WO 9201047 A
- WO 9220791 A
- WO 9306213 A
- WO 9311236 A
- WO 9319172 A
- WO 9501438 A
- WO 9515388 A
- WO 9219759 A
- WO 9814580 A
- WO 9813388 A
- WO 9951743 A
- WO 2006006693 A
- WO 2009072604 A
- WO 2012067176 A
- WO 0505636 A
- WO 0233072 A
- WO 0166737 A
- WO 2009041621 A
- WO 9012874 A
- WO 2010064697 A1
- WO 2016167263 A1

**Non-patent literature cited in the description**

- **ABDUL-FATTAH et al.** Investigating factors leading to fogging of glass vials in lyophilized drug products. *EUROPEAN JOURNAL OF PHARMACEUTICS AND BIOPHARMACEUTICS,* 01 January 2013, vol. 85, 314-326
- **KOHLER. G. ; MILSTEIN, C.** *Methods Enzymol.,* 1981, vol. 73, 3-46
- **CARL ; A. K. BORREBAECK ; JAMES, W. LARRICK.** THERAPEUTIC MONOCLONAL ANTIBODIES. MACMILLAN PUBLISHERS LTD, 1990
- **SATO, K. et al.** *Cancer Res.,* 1993, vol. 53, 851-856
- **TSURUSHITA N ; HINTON PR ; KUMAR S.** Design of humanized antibodies: from anti-Tac to Zenapax. *Methods,* May 2005, vol. 36 (1), 69-83
- **RAJPAL A ; BEYAZ N ; HABER L ; CAPPUCCILLI G ; YEE H ; BHATT RR ; TAKEUCHI T ; LERNER RA ; CREA R.** A general method for greatly improving the affinity of antibodies by using combinatorial libraries. *Proc Natl Acad Sci U.S.A.,* 14 June 2005, vol. 102 (24), 8466-71
- **EWERT S ; HONEGGER A ; PLUCKTHUN A.** Stability improvement of antibodies for extracellular and intracellular applications: CDR grafting to stable frameworks and structure-based framework engineering. *Methods,* October 2004, vol. 34 (2), 184-99
- **KIM SJ ; PARK Y ; HONG HJ.** Antibody engineering for the development of therapeutic antibodies. *Mol Cells,* 31 August 2005, vol. 20 (1), 17-29
- **HINTON PR ; XIONG JM ; JOHLFS MG ; TANG MT ; KELLER S ; TSURUSHITA N.** An engineered human IgG1 antibody with longer serum half-life. *J Immunol.,* 01 January 2006, vol. 176 (1), 346-56
- **GHETIE V ; POPOV S ; BORVAK J ; RADU C ; MATESOI D ; MEDESAN C ; OBER RJ ; WARD ES.** Increasing the serum persistence of an IgG fragment by random mutagenesis. *Nat Biotechnol.,* vol. 15 (7), 637-40
- *Farmaco.,* 30 August 1999, vol. 54 (8), 497-516
- *Cancer J.,* May 2008, vol. 14 (3), 154-69
- **CLELAND et al.** *Crit. Rev. Ther. Drug Carrier Syst.,* 1993, vol. 10 (4), 307-77
- **WANG.** *Int. J. Pharm.,* 1999, vol. 185 (2), 129-88
- **WANG.** *Int. J. Pharm,* 2000, vol. 203 (1-2), 1-60
- **CHI et al.** *Pharm. Res.,* 2003, vol. 20 (9), 1325-36